# EUROPÄISCHE PATENTSCHRIFT

(11) **EP 3 094 614 B1**
(45) Veröffentlichungstag und Bekanntmachung des Hinweises auf die Patenterteilung: **02.05.2018**
(21) Anmeldenummer: 15702396.1
(22) Anmeldetag: 16.01.2015
(51) Int. Cl.: C07C 41/18

(54) **VERFAHREN ZUR HERSTELLUNG VON ARYLPROPENEN**
METHOD FOR PRODUCING ARYLPROPENES
PROCÉDÉ DE FABRICATION D'ARYLPROPÈNES

(30) Priorität: 17.01.2014 EP 14151641
(43) Veröffentlichungstag der Anmeldung: 23.11.2016
(73) Patentinhaber: BASF SE, 67056 Ludwigshafen am Rhein (DE)
(72) Erfinder: RÜDENAUER, Stefan, 69469 Weinheim (DE); LANVER, Andreas, 68199 Mannheim (DE); PELZER, Ralf, 37699 Fürstenberg (DE); EBEL, Klaus, 68542 Heddesheim (DE); FENLON, Thomas, 68165 Mannheim (DE)
(74) Vertreter: BASF IP Association
(86) Internationale Anmeldenummer: PCT/EP2015/050792
(87) Internationale Veröffentlichungsnummer: WO 2015/107156

(56) Entgegenhaltungen:
- DE-B1- 2 418 974
- US-A1- 2008 045 752

## Beschreibung

Die vorliegende Erfindung betrifft ein Verfahren zur Herstellung von Arylpropenen, insbesondere 1-Methoxy-4-(1-propenyl)-benzol (Anethol), mittels Thermolyse der entsprechenden 1,1-Diarylpropane.

### HINTERGRUND DER ERFINDUNG

Anethol (1-Methoxy-4-(1-propenyl)-benzol) ist aufgrund seines charakteristischen Anisgeruchs als Duft- und Aromastoff von großem kommerziellen Interesse. Insbesondere wird Anethol als Duftstoff in Wasch- und Reinigungsmitteln oder Kosmetika eingesetzt sowie als Aromastoff in der Lebensmittelindustrie verwendet.

Die Herstellung von Anethol ist seit langem im Stand der Technik bekannt. So lässt sich Anethol unter anderem aus natürlichen Quellen gewinnen, z. B. aus Fenchelöl oder Anisöl - siehe RU2068920 und CN102491884.

Allerdings ist die Gewinnung von Duftstoffen aus natürlichen Quellen meist teuer und die so erhältlichen Mengen limitiert. Zudem variiert die Reinheit oder Produktionsmenge dieser Duftstoffe oft aufgrund wechselhafter Umweltbedingungen bei der Produktion der Rohstoffe aus denen diese isoliert werden. Daher besteht ein großes Interesse, Duftstoffe aus natürlichen Quellen zumindest teilweise durch synthetisch erhältliche Substanzen zu ersetzen.

Im Stand der Technik sind diverse synthetische Verfahren zur Herstellung von Anethol bekannt. Beispielsweise beschreibt Bauer et al., Common Fragrance and Flavor Materials, 2001, 4th Edition, Wiley-VHC, die Herstellung von Anethol durch eine basenkatalysierte Umlagerung von 1-Methoxy-4-allyl-benzol (Estragiol).

Eine weitere im Stand der Technik bekannte Methode zur Herstellung von Anethol, wie sie beispielsweise in der SU261380 und SU355144 beschrieben ist, beinhaltet eine Friedel-Crafts-Acylierung von Anisol mit Propionsäurehalogeniden oder Propionsäureanhydrid, gefolgt von der Reduktion der Carbonylgruppe und anschließender Eliminierung von Wasser. Die Acylierung von Anisol mit Propionsäureanhydrid kann unter anderem mit ZnCl₂ und FeCl₃ erfolgen (siehe Maslozhirovaya Promyshlennost (1974), Band 9, Seiten 29-30).

CN103058835 beschreibt ein Verfahren zur Synthese von Anethol bei dem eine Friedel-Crafts-Reaktion ausgehend von Anisol und Propionsäurechlorid durchgeführt wird, gefolgt von einer Reduktion der Carbonylgruppe zum entsprechenden Alkohol mit Hilfe von NaBH₄ und anschließender Eliminierung von Wasser mit p-TsOH und KHSO₄.

DE 2418974 B1 beschreibt ein Verfahren zur Herstellung von Anethol, bei dem in einem ersten Schritt Anisol mit Propionaldehyd in Gegewart von sauren Katalysatoren zu einer Mischung aus Bis-(methoxyphenyl)-propanen kondensiert wird. In einem zweiten Schritt werden die Kondensationsprodukte duch Einsatz katalytischer Mengen Säure und durch Erhitzen auf Temperaturen von 100 bis 300°C in flüssiger Phase in p-Anethol, bzw. o-Anethol und Anisol gespalten. Konkret wird die Spaltung der Bis-(methoxyphenyl)-propane in Gegenwart einer katalytischen Menge konzentrierter Phosphorsäure bei 200°C durchgeführt, wobei die sich bildenden Spaltprodukte bei etwa 5 bis 30 Torr abdestilliert werden. Reines p-Anethol wird aus dem Destillat durch fraktionierte Destillation gewonnen. Von Nachteil ist, dass die Bis-(methoxyphenyl)-propane nicht vollständig gespalten werden, d.h. nur partiell umgesetzt werden. Zudem werden nur moderate Ausbeuten an Anetholen erreicht. Die lange Verweilzeit der Bis-(methoxyphenyl)-propane und der Spaltprodukte bei erhöhten Temperaturen führt zudem zu einer vermehrten Bildung von Nebenprodukten, ungewollter Isomere sowie Oligomere und Polymere.

### ZUSAMMENFASSUNG DER ERFINDUNG

Der vorliegenden Erfindung liegt die Aufgabe zugrunde, ein verbessertes Verfahren zur Herstellung von Arylpropenen, insbesondere von Anethol, bereitzustellen mit dem die zuvor genannten Nachteile vermieden werden können. Zudem sollte das Verfahren einfach und effizient sein, um eine kostengünstige Herstellung von Arylpropenen zu ermöglichen.

Diese Aufgabe wird überaschenderweise gelöst durch ein Verfahren zur Herstellung von Arylpropenen der allgemeinen Formel (I) 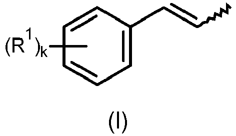 worin
- k: die Werte 0, 1, 2 oder 3 aufweist und
- R¹: unabhängig voneinander ausgewählt ist unter C₁-C₆-Alkoxy, Di-(C₁-C₆-alkyl)-aminen und Hydroxy,
umfassend die Thermolyse wenigstens einer 1,1-Diarylpropan-Verbindung der allgemeinen Formel (II) 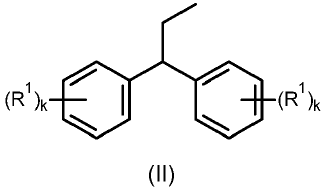 worin k und R¹ die obengenannten Bedeutungen aufweisen,
wobei man die Verbindung der Formel II in der Gasphase in einer Reaktionszone mit wenigstens einem festen, sauren, oxidischen Katalysator in Kontakt bringt.

Aufgrund der Reaktionsführung des Verfahrens in der Gasphase bei hohen Temperaturen kann die Verweilzeit der Einsatzstoffe und der Spaltprodukte in der Reaktionszone sehr kurz gehalten werden. Die Bildung von Nebenprodukten lässt sich somit stark reduzieren. Mit dem erfindungsgemäßen Verfahren kann die Bildung unerwünschten Isomeren gering gehalten werden, was zu guten Produktselektivitäten führt. Trotz der geringen Verweilzeit der Einsatzstoffe in der Reaktionszone können mit dem erfindungsgemäßen Verfahren hohe Umsätze erzielt werden. Das erfindungsgemäße Verfahren kann kontinuierlich durchgeführt werden und zeichnet sich durch seine Einfachheit und Wirtschaftlichkeit aus.

Die Arylpropene der allgemeinen Formel I lassen sich mit dem erfindungsgemäßen Verfahren aus leicht zugänglichen Ausgangsmaterialien herstellen, wodurch diese problemlos in großtechnischem Maßstab bereitgestellt werden können.

### AUSFÜHRLICHE BESCHREIBUNG DER ERFINDUNG

Im Rahmen der vorliegenden Erfindung umfasst der Ausdruck "C₁-C₆-Alkoxy" geradkettige oder verzweigte C₁-C₆-Alkoxygruppen, umfassend 1 bis 6 Kohlenstoffatome, bevorzugt 1 bis 4 Kohlenstoffatome, die über ein Sauerstoffatom gebunden sind. Dazu zählen Methoxy, Ethoxy, n-Propoxy, 1-Methylethoxy, n-Butoxy, 1-Methylpropoxy, 2-Methylpropoxy, 1,1-Dimethylethoxy, n-Pentoxy, 1-Methylbutoxy, 2-Methylbutoxy, 3-Methylbutoxy, 1,1-Dimethylpropoxy, 1,2-Dimethylpropoxy, 2,2-Dimethylpropoxy, 1-Ethylpropoxy, n-Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy, 1-Ethyl-2-methylpropoxy und dergleichen. Insbesondere umfasst C₁-C₆-Alkoxy geradkettige oder verzweigte Alkyloxygruppen mit 1, 2 oder 3 C-Atomen (C₁-C₃-Alkoxy).

Im Rahmen der vorliegenden Erfindung bezieht sich der Ausdruck "Di-(C₁-C₆-alkyl)-amin" auf Alkylamine die mit zwei C₁-C₆-Alkylresten substituiert sind. Die C₁-C₆-Alkylreste umfassen jeweils unabhängig voneinander geradkettige oder verzweigte C₁-C₆-Alkylgruppen. Dazu zählen Methyl, Ethyl, Propyl, Isopropyl, n-Butyl, Isobutyl, sec.-Butyl, tert.-Butyl, n-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-2-methylpropyl und dergleichen. Insbesondere umfassen die C₁-C₆-Alkylreste unabhängig voneinander geradkettige oder verzweigte Alkylgruppen mit 1, 2 oder 3 C-Atomen (C₁-C₃-Alkyl).

In bevorzugten Ausführungsformen steht in den im erfindungsgemäßen Verfahren verwendeten Verbindungen der allgemeinen Formel I und II k für 1 und der Rest R¹ für Hydroxy oder C₁-C₆-Alkoxy, insbesondere für Hydroxy oder C₁-C₄-Alkoxy und speziell für Hydroxy oder Methoxy.

Insbesondere umfassen die im erfindungsgemäßen Verfahren eingesetzten Verbindungen der allgemeinen Formel II zu wenigstens 50 mol-%, insbesondere zu wenigstens 80 mol-%, bezogen auf die Gesamtmenge der Verbindungen der Formel II, Verbindungen der allgemeinen Formel (II.a), 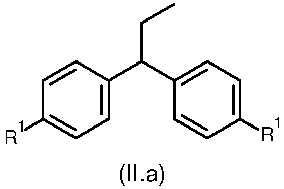 worin beide R¹ gleich sind und die zuvor genannten Bedeutungen aufweisen und insbesondere für Hydroxy oder C₁-C₄-Alkoxy und speziell für Hydroxy oder Methoxy stehen. Neben der Verbindung der allgemeinen Formel II.a können die Verbindungen der Formel II insbesondere auch Regioisomere von II.a umfassen, also Verbindungen der Formel I mit k = 1, in denen die Reste R¹ in der 2- oder 3-Position, bezogen auf die Bindungsstelle der Phenylringe stehen.

In einer besonders bevorzugten Ausführungsform umfassen die im erfindungsgemäßen Verfahren eingesetzten Verbindungen der allgemeinen Formel II zu wenigstens 50 mol-%, insbesondere zu wenigstens 80 mol-%, bezogen auf die Gesamtmenge der Verbindungen der Formel II, 1,1-Bis(4-methoxyphenyl)propan und 1,1-Bis(4-hydroxyphenyl)propan.

In dem erfindungsgemäßen Verfahren werden die 1,1-Diarylpropan-Verbindung der allgemeinen Formel II in der Gasphase in der Reaktionszone mit wenigstens einem festen, sauren, oxidischen Katalysator in Kontakt gebracht, wodurch diese zu den Arylpropene der allgemeinen Formel I gespalten werden.

Die mit dem erfindungsgemäßen Verfahren hergestellten Arylpropene der allgemeinen Formel I liegen im Allgemeinen als cis/trans-Isomerengemische sowie auch als Regioisomerengemische, wie beispielsweise Mischungen aus ortho- und para-substituierten Verbindungen, vor. So wird beispielsweise bei der thermischen Spaltung des Einsatzstoffes 1,1-Bis(4-methoxyphenyl)propan neben dem gewünschten p-trans-Anethol, auch das cis-Isomer (p-cis-Anethol) sowie ein cis/trans-Isomerengemisch des entsprechenden ortho-Substituierten Anethols (o-cis-Anethol, bzw. o-trans-Anethol) gebildet. Als weiteres Spaltprodukt wird ebenfalls Anisol gebildet.

Im Rahmen der vorliegenden Erfindung wird diese Spaltung auch als Thermolyse oder thermische Spaltung bezeichnet.

Die thermische Spaltung kann in der Regel bei Umgebungsdruck oder vermindertem oder erhöhtem Druck erfolgen. Bevorzugt wird die thermische Spaltung bei Umgebungsdruck oder vermindertem Druck durchgeführt, z. B. bei einem Druck im Bereich von 0,1 bis 2,0 bar, insbesondere in einem Bereich von 0,8 bis 1,1 bar.

Im Rahmen der vorliegenden Erfindung bedeutet der Ausdruck "in der Gasphase", dass die Thermolyse bei einer Temperatur durchgeführt wird, bei der sich der größte Teil, z. B. wenigstens 99%, der eingesetzten 1,1-Diarylpropan-Verbindungen der allgemeinen Formel II in der Gasphase befindet. Idealerweise liegt die Temperatur in der Reaktionszone mindestens 5°C, insbesondere 10°C, speziell 15°C, z. B. 15 bis 30°C über der entsprechenden Siedetemperatur der eingesetzten 1,1-Diarylpropan-Verbindungen der allgemeinen Formel II bei dem in der Reaktionszone vorliegenden Druck.

In dem erfindungsgemäßen Verfahren liegt die Temperatur in der Reaktionszone üblicherweise im Bereich von 250°C bis 650°C. Bevorzugt liegt die Temperatur in der Reaktionszone im Bereich von 300°C bis 600°C, besonders bevorzugt im Bereich von 320°C bis 550°C.

Die thermische Spaltung kann in Abwesenheit oder Anwesenheit eines Lösungsmittels LM erfolgen. In einer bevorzugten Ausführungsform wird der Reaktionszone zusammen mit der Verbindung der Formel II zusätzlich wenigstens ein Lösungsmittel LM zugeführt.

Falls die thermische Spaltung in Gegenwart wenigstens eines Lösungsmittels LM durchgeführt wird, handelt es sich dabei in der Regel um inerte Lösungsmittel mit einem Siedepunkt unter der gewählten Reaktionstemperatur, z. B. im Bereich von 30°C bis 200°C, insbesondere im Bereich von 60 bis 150°C. Im Rahmen der vorliegenden Erfindung ist unter einem inerten Lösungsmittel ein Lösungsmittel zu verstehen, welches unter den gewählten Reaktionsbedingungen keine Reaktion mit den an der Spaltung beteiligten Verbindungen eingeht.

Geeignete inerte Lösungsmittel sind beispielsweise aliphatische Kohlenwasserstoffe, halogenierte aliphatische Kohlenwasserstoffe, aromatische und substituierte aromatische Kohlenwasserstoffe, Ether, Alkylnitrile sowie Alkanole oder Wasser und deren Gemische. Bevorzugt ist das Lösungsmittel LM ausgewählt unter aliphatischen Kohlenwasserstoffen, wie Pentan, Hexan, Heptan, Ligroin, Petrolether oder Cyclohexan, halogenierten Kohlenwasserstoffen, wie Dichlormethan, Trichlormethan, Tetrachlormethan, aromatischen Kohlenwasserstoffen, wie Benzol, Toluol, Xylol, halogenierten aromatischen Kohlenwasserstoffen, wie Chlorbenzol, Dichlorbenzolen, Ethern, wie Diethylether, Methyl-tert.-Butylether, Dibutylether, Tetrahydrofuran oder Dioxan, C₁-C₄-Alkylnitrilen, wie Acetonitril oder Propionitril, Alkanolen, wie Methanol, Ethanol, Wasser und Mischungen davon. Besonders bevorzugt ist das Lösungsmittel LM ausgewählt unter Diethylether, Methyl-tert-butylether, Tetrahydrofuran, Acetonitril oder Wasser. Besonders bevorzugt umfasst das Lösungsmittel LM zu wenigstens 0,3 Gew.-% bezogen auf die Gesamtmenge an eingesetztem Lösungsmittel LM, Wasser. Insbesondere ist das Lösungsmittel LM Wasser.

Falls die thermische Spaltung in Gegenwart wenigstens eines Lösungsmittels LM durchgeführt wird, liegt das Masseverhältnis von Lösungsmittel LM zur Verbindung der Formel II im Bereich von 20 : 1 bis 1 : 100. Bevorzugt liegt das Masseverhältnis von Lösungsmittel LM zur Verbindung der Formel II im Bereich von 10 : 1 bis 1 : 50. Besonders bevorzugt liegt das Masseverhältnis von Lösungsmittel LM zur Verbindung der Formel II im Bereich von 2 : 1 bis 1 : 50.

Bei dem im erfindungsgemäßen Verfahren eingesetzten festen, oxidischen Katalysator handelt es sich um saure feste, oxidische Materialien.

Geeignete feste, oxidische Materialien sind beispielsweise Metalloxide und Halbmetalloxide des Aluminiums, Magnesiums, Zinks, Titans, Zirkons, Hafniums, Siliziums, Cers oder Lanthans, Mischoxide dieser Metalle sowie Oxid-Gemische. Beispiele für solche Oxide sind Aluminiumoxid, Magnesiumoxid, Titandioxid, Zink(IV)oxid, Ceroxid, Lanthanoxid und Siliziumdioxid sowie Mischungen davon. Bevorzugt handelt es sich bei dem im erfindungsgemäßen Verfahren eingesetzten festen, oxidischen Katalysator um Siliziumdioxid, insbesondere um Kieselsäure.

Bevorzugte oxidische Katalysatoren weisen eine spezifische BET-Oberfläche, bestimmt mittels Stickstoffadsorbtion bei 77 K gemäß DIN ISO 9277 2003-5, im Bereich von 100 bis 500 m2/g auf.

Vorzugsweise handelt es sich bei dem Katalysator um ein saures Metalloxid oder Halbmetalloxid oder um ein Metall- oder Halbmetalloxid, das mit einer anorganischen Säure, beispielsweise mit Salpetersäure, Schwefelsäure oder Phosphorsäure imprägniert wurde.

In einer besonders bevorzugten Ausführungsform des erfindungsgemäßen Verfahrens handelt es sich bei dem festen, oxidischen Katalysator um eine Kieselsäure, die gegebenenfalls mit einer oder mehreren anorganischen Säuren, beispielsweise mit Salpetersäure, Schwefelsäure oder Phosphorsäure, imprägniert ist.

Der Katalysator kann ein Pulver, ein regelmäßig oder unregelmäßig geformtes Granulat, beispielsweise Katalysatorstränge, oder ein Formkörper sein. Bevorzugt wird der Katalysator in Form von Strängen eingesetzt. Gewöhnlich weisen die Katalysatorstränge einen Durchmesser im Bereich von 0,5 bis 10 mm, insbesondere im Bereich von 0,5 bis 3 mm, auf.

Bevorzugt wird das erfindungsgemäßen Verfahrens in kontinuierlicher Fahrweise durchgeführt, wobei die Verbindung der Formel II kontinuierlich durch die Reaktionszone, welche den festen oxidischen Katalysator enthält, geführt wird.

Dabei wird die Verbindung der Formel II in der Regel mit einer Katalysatorbelastung von 0,01 bis 5 kg Verbindung II pro kg Katalysator und Stunde, vorzugsweise von 0,02 bis 2 kg Verbindung II pro kg Katalysator und Stunde und insbesondere mit 0,05 bis 1 kg Verbindung II pro kg Katalysator und Stunde, durch die Reaktionszone geführt.

Bevorzugt wird die Verbindung II mit Hilfe eines Trägergases der Reaktionszone zugeführt. Als Trägergas wird bevorzugt ein inertes Gas eingesetzt. Im Rahmend der vorliegenden Erfindung ist unter einem inerten Gas ein Gas zu verstehen, welches unter den gewählten Reaktionsbedingungen keine Reaktion mit den an der Spaltung beteiligten Verbindungen eingeht.

Bevorzugt wird in dem erfindungsgemäßen Verfahren als Trägergas He, Ar oder N₂ eingesetzt, insbesondere N₂.

Das Volumenstromverhältnis des Trägergases zu den Einsatzstoffen (Edukte und LM) liegt üblicherweise im Bereich von 10:1 bis 10000:1, bevorzugt im Bereich von 100:1 bis 5000:1 und insbesondere im Bereich von 800:1 bis 4000:1.

In einer bevorzugten Ausführungsform des erfindungsgemäßen Verfahrens werden die Verbindungen der Formel II der Reaktionszone kontinuierlich zugeführt und das Umsetzungsprodukt unmittelbar nach Verlassen der Reaktionszone abgeschreckt. Die Abschreckung erfolgt im Allgemeinen durch einen nachgeschalteten Kühler. Der Kühler steht üblicherweise in direkter Verbindung mit der Reaktionszone.

Die Reaktionszone kann in den für Gasphasenreaktionen üblichen Formen ausgestaltet sein. In der Regel handelt es sich bei der Reaktionszone um eine zylindrische Reaktoranordnung, beispielsweise ein Reaktionsrohr, die mit dem Katalysator befüllt ist. Die Befüllung kann ein Festbett, ein Fließbett oder eine Packung sein. Die Reaktionszone kann stehend oder liegend angeordnet sein. Bei einer stehenden Anordnung kann die Verbindung der Formel II aufsteigend oder absteigend durch die Reaktionszone geführt werden. Bevorzugt ist die Reaktionszone rohrförmig ausgestaltet, wobei das Verhältnis von Länge zu Innendurchmesser wenigstens 3 : 1 beträgt. Bevorzugt liegt das Verhältnis von Länge zu Innendurchmesser im Bereich von 3 : 1 bis 100 : 1, insbesondere im Bereich von 5:1 bis 10:1.

Die Reaktoranordnung, welche die Reaktionszone umfasst, weist üblicherweise Mittel zum Beheizen der Reaktionszone auf, beispielsweise eine elektrische Heizung oder eine Induktionsheizung.

Vorzugsweise ist der Reaktionszone einer Verdampferanordnung vorgeschaltet, bei der die Verbindung der Formel II und gegebenenfalls das Lösungsmittel verdampft und so in die Gasphase gebracht werden.

Vorzugsweise ist unmittelbar im Anschluss an die Reaktionszone wenigstens ein Mittel zum Abschrecken des Reaktionsprodukts, beispielsweise ein Intensivkühler oder eine Kühlfalle, nachgeschaltet.

Die nach dem erfindungsgemäßen Verfahren hergestellten und in der Regel in kondensierter Form vorliegenden Umsetzungsprodukte können nach üblichen, dem Fachmann einschlägig bekannten Methoden, wie beispielsweise Extraktion, Destillation, Kristallisation oder chromatographische Trennverfahren, gereinigt werden.

Wie eingangs erwähnt, liegen die mit dem erfindungsgemäßen Verfahren hergestellten Arylpropene der allgemeinen Formel I im Allgemeinen als cis/trans-Isomerengemische sowie auch als Regioisomerengemische, wie beispielsweise Mischungen aus ortho- und para-substituierten Verbindungen, vor. Die Auftrennung der einzelnen Isomere gelingt in der Regel durch fraktionierte Destillation oder durch chromatographische Auftrennung.

Bevorzugt werden die Umsetzungsprodukte einer destillativen Auftrennung unterzogen. Geeignete Vorrichtungen zur destillativen Auftrennung umfassen Destillationskolonnen, wie Bodenkolonnen, die mit Glockenböden, Siebplatten, Siebböden, Packungen oder Füllkörpern u. ausgerüstet sein können, oder Drehbandkolonnen Verdampfer, wie Dünnschichtverdampfer, Fallfilmverdampfer, Zwangsumlaufverdampfer, Sambay-Verdampfer, etc. und Kombinationen davon. Besonders bevorzugt wird die zur destillativen Auftrennung der Umsetzungsprodukte Destillationskolonnen verwendet, insbesondere Drehbandkolonnen.

Die in dem erfindungsgemäßen Verfahren zur Herstellung von Verbindungen der allgemeinen Formel I als Ausgangsstoff benötigten 1,1-Diarylpropane der allgemeinen Formel II können entweder kommerziell erworben oder nach literaturbekannten Synthesewegen hergestellt werden, wie sie beispielsweise aus DE19742418974 bekannt sind.

Die Erfindung wird anhand der im Folgenden beschriebenen Beispiele näher erläutert. Dabei sollen die Beispiele nicht als einschränkend für die Erfindung verstanden werden.

In den nachfolgenden Beispielen werden folgende Abkürzungen verwendet:
Dimer 1: 1,1-Bis(4-methoxyphenyl)propan

### BEISPIELE

### I) Herstellungsbeispiele:

### Herstellung von 1-Methoxy-4-(1-propenyl)-benzol (Anethol) aus 1,1-Bis(4-methoxyphenyl)propan (Beispiele 1-6)

Es wurden mehrere Versuche zur Spaltung von 1,1-Bis(4-methoxyphenyl)propan (Dimer1) in Anisol und Anethol durchgeführt. Als Reaktor wurde eine Gasphasenofen mit einem Innendurchmesser von 4 cm und elektrischer Heizung verwendet. Dieser wurde zunächst auf einer Strecke von 15 cm mit Quarzringen befüllt und anschließend auf einer Strecke von 20 cm mit Katalysatorsträngen mit einem Strangdurchmesser von 3 mm bestückt. Der mit Quarzringen befüllte Teil des Gasphasenofens dient dabei als Verdampferstrecke für das in Rieselfahrweise zugefahrene 1,1-Bis(4-methoxyphenyl)propan sowie das gegebenenfalls zugesetzte Lösungsmittel. Als Trägergas wurde Stickstoff verwendet. Das Reaktionsprodukt wurde bei einer Temperatur von 5°C im Sumpf und einem nachgeschalteten Kühler kondensiert. Bei der Verwendung eines Lösungsmittels, in diesem Fall Wasser, wurde dieses zusammen mit 1,1-Bis(4-methoxyphenyl)propan in zwei getrennten Zuläufen auf die Verdampferstrecke gefahren. Die Analyse der organischen Produkte erfolgte in den Experimenten nach Abtrennung und Verwerfung der wässrigen Phase.

**Tabelle 1**

| Katalysator und Reaktionsbedingungen der Beispiele 1-12 | | | | | | |
|---|---|---|---|---|---|---|
| Beispiel | Katalysator | T [°C] | H₂O [ml/h] | Dimer1 [ml/h] | Belastung [g Dimer1 / g Kat. / h] | N₂ [l/h] |
| 1 | SiO₂ x H₃PO₄ | 400 | - | 33,0 | 0,500 | 9 |
| 2 | SiO₂ x H₃PO₄ | 450 | - | 32,5 | 0,490 | 10 |
| 3 | SiO₂ x H₃PO₄ | 500 | - | 23,0 | 0,350 | 20 |
| 4 | SiO₂ | 450 | - | 16,0 | 0,240 | 20 |
| 5 | SiO₂ | 400 | 48,0 | 15,5 | 0,235 | 9 |
| 6 | SiO₂ | 400 | 23,2 | 6,4 | 0,097 | 9 |
| 7 | SiO₂ x H₃PO₄ | 350 | 34,2 | 9,9 | 0,26 | 20 |
| 8 | SiO₂ x H₃PO₄ | 400 | 33,2 | 10,6 | 0,28 | 20 |

**Tabelle 2**

| Eduktumsatz sowie Ausbeuten und Selektivitäten der gebildeten Spaltprodukte | | | | | | | |
|---|---|---|---|---|---|---|---|
| Beispiel | Umsatz Dimer1 [%] | Anisol | | Anethol Isomere | | p-trans-Anethol | |
| | | Ausbeute [%] | Selektivität [%] | Ausbeute [%] | Selektivität [%] | Ausbeute [%] | Selektivität [%] |
| 1 | 46,3 | 26,7 | 57,6 | 29,8 | 64,4 | 20,3 | 43,9 |
| 2 | 61,0 | 30,6 | 50,2 | 28,7 | 47,0 | 18,6 | 30,5 |
| 3 | 46,1 | 2,9 | 6,3 | 0,9 | 1,8 | 0,5 | 1,2 |
| 4 | 77,1 | 41,0 | 53,2 | 17,3 | 22,4 | 8,9 | 11,5 |
| 5 | 84,9 | 77,3 | 91,0 | 73,6 | 86,7 | 45,1 | 53,1 |
| 6 | 84,5 | 68,3 | 80,8 | 69,1 | 81,8 | 42,8 | 50,6 |
| 7 | 90,5 | - | - | 42,7 | 47,2 | 27,2 | 30,6 |
| 8 | 40,8 | - | - | 31,6 | 77,5 | 22,2 | 54,4 |

### Vergleichsbeispiel V9:

Als Vergleich wurde die thermische Spaltung von 1,1-Bis(4-methoxyphenyl)propan in Anethol und Anisol gemäß dem in DE19742418974 beschriebenen Verfahren nachgestellt. Dazu wurden 100 g 1,1-Bis(4-methoxyphenyl)propan in einem Glaskolben mit aufgesetzter Destillationvorrichtung vorgelegt, auf 200°C erhitzt und 0,5 g konzentrierte Phosphorsäure zugesetzt. Während des Reaktionsverlaufs (mind. 15 Min.) wurden die gebildeten Produkte unter vermindertem Druck abdestilliert. Man erhielt bei einem Umsatz von 67,3% eine Gesamtausbeute an Anetholen (alle Isomere) von 57,3% mit einer Ausbeute an p-trans-Anethol von 41,6%.

### II) Destillation:

Eine repräsentativer Reaktionsaustrag mit einem Gehalt an p-trans-Anethol von 16,2 Gew.-% (mittels GC bestimmt) wurde mit Hilfe einer Batch-Destillation in einer 100 cm langen, schutzbeheizten Drehkolonne (ca. 20 theoretische Trennstufen), bei einem Druck am Kopf der Kolonne von 20 mbar und zum Ende von 2 mbar, reindestilliert. Das Rücklaufverhältnis betrug bis zur Abtrennung von Anisol 10:1 und wurde auf 15:1 erhöht, um eine höhere Reinheit der Produktfraktion zu erzielen. Die maximale Sumpftemperatur betrug 165°C. Als Hauptfraktion wurden zwei Fraktionen mit einem Gehalt an p-trans-Anethol von 97,6 Gew.-% (GC-Gehalt) und 96,5 Gew.-% (GC-Gehalt) erhalten. Beide entsprachen dem charakteristischen Geruchsbild.

## Patentansprüche

1. Verfahren zur Herstellung von Arylpropenen der allgemeinen Formel (I) 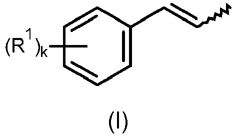 worin
k die Werte 0, 1, 2 oder 3 aufweist und
R¹ unabhängig voneinander ausgewählt ist unter C₁-C₆-Alkoxy, Di-(C₁-C₆-alkyl)-aminen und Hydroxy,
umfassend die Thermolyse wenigstens einer 1,1-Diarylpropan-Verbindung der allgemeinen Formel (II) 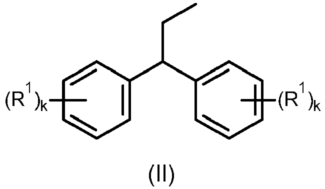 worin k und R¹ die obengenannten Bedeutungen aufweisen,
wobei man die Verbindung der Formel II in der Gasphase in einer Reaktionszone mit wenigstens einem festen, sauren, oxidischen Katalysator in Kontakt bringt.

2. Verfahren nach Anspruch 1, wobei in den Formeln I und II k = 1 ist und R¹ für Hydroxy oder C₁-C₆-Alkoxy und speziell für Hydroxy oder Methoxy steht.

3. Verfahren nach Anspruch 1 oder 2, wobei die 1,1-Diarylpropan-Verbindungen (II) zu wenigstens 50 mol-%, bezogen auf die Gesamtmenge der Verbindungen der Formel II, Verbindungen der allgemeinen Formel (II.a) umfassen, 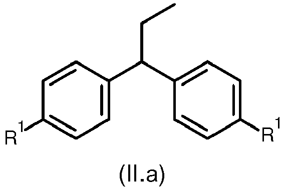 worin beide R¹ gleich sind und die zuvor genannten Bedeutungen aufweisen.

4. Verfahren nach einem der vorhergehenden Ansprüche, wobei der Reaktionszone zusammen mit der Verbindung der Formel II zusätzlich wenigstens ein Lösungsmittel LM zugeführt wird.

5. Verfahren nach Anspruch 4, wobei es sich bei dem Lösungsmittel LM um Wasser handelt.

6. Verfahren nach Anspruch 4 oder 5, wobei das Masseverhältnis von Lösungsmittel LM zur Verbindung der Formel II im Bereich von 20 : 1 bis 1 : 100 liegt.

7. Verfahren nach einem der vorhergehenden Ansprüche, wobei der Katalysator Siliziumdioxid umfasst.

8. Verfahren nach Anspruch 7, wobei der Katalysator eine Kieselsäure ist, die gegebenenfalls mit einer oder mehreren anorganischen Säuren imprägniert ist.

9. Verfahren nach einem der vorhergehenden Ansprüche, wobei die Verbindung der Formel II mit dem Katalysator bei einer Temperatur im Bereich von 250 bis 650°C in Kontakt gebracht wird.

10. Verfahren nach einem der vorhergehenden Ansprüche, wobei man die Verbindung der Formel II kontinuierlich durch die Reaktionszone, welche den festen oxidischen Katalysator enthält, führt.

11. Verfahren nach Anspruch 10, wobei man die Verbindung der Formel II der Reaktionszone kontinuierlich zuführt und das Umsetzungsprodukt unmittelbar nach Verlassen der Reaktionszone abschreckt.

12. Verfahren nach Anspruch 10 oder 11, wobei man die Verbindung der Formel II mit Hilfe eines Trägergases der Reaktionszone zuführt.

13. Verfahren nach einem der Ansprüche 10 bis 12, wobei man die Verbindung der Formel II mit einer Katalysatorbelastung von 0,01 bis 5 kg Verbindung II pro kg Katalysator und Stunde durch die Reaktionszone führt.

## Claims

1. A process for preparing arylpropenes of the general formula (I) 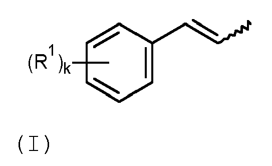 in which
k has the values of 0, 1, 2 or 3 and
R¹ is independently selected from C₁-C₆-alkoxy, di(C₁-C₆-alkyl)amines and hydroxyl,
comprising the thermolysis of at least one 1,1-diarylpropane compound of the general formula (II) 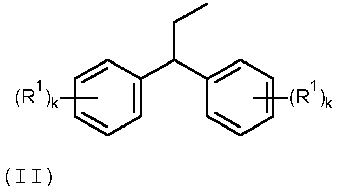 in which k and R¹ have the definitions given above, wherein the compound of the formula II in the gas phase is contacted with at least one solid acidic oxidic catalyst in a reaction zone.

2. The process according to claim 1, wherein, in the formulae I and II, k = 1 and R¹ is hydroxyl or C₁-C₆-alkoxy and specifically hydroxyl or methoxy.

3. The process according to claim 1 or 2, wherein the 1,1-diarylpropane compounds (II) comprise, to an extent of at least 50 mol%, based on the total amount of the compounds of the formula II, compounds of the general formula (II.a) 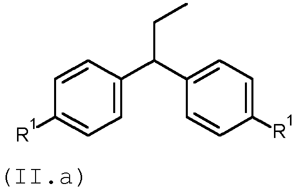 in which both R¹ are the same and have the definitions given above.

4. The process according to any of the preceding claims, wherein the reaction zone is supplied, together with the compound of the formula II, additionally with at least one solvent S.

5. The process according to claim 4, wherein the solvent S is water.

6. The process according to claim 4 or 5, wherein the mass ratio of solvent S to the compound of the formula II is in the range from 20:1 to 1:100.

7. The process according to any of the preceding claims, wherein the catalyst comprises silicon dioxide.

8. The process according to claim 7, wherein the catalyst is a silica which has optionally been impregnated with one or more inorganic acids.

9. The process according to any of the preceding claims, wherein the compound of the formula II is contacted with the catalyst at a temperature in the range from 250 to 650°C.

10. The process according to any of the preceding claims, wherein the compound of the formula II is conducted continuously through the reaction zone comprising the solid oxidic catalyst.

11. The process according to claim 10, wherein the compound of the formula II is supplied continuously to the reaction zone and the reaction product is quenched immediately after leaving the reaction zone.

12. The process according to claim 10 or 11, wherein the compound of the formula II is supplied to the reaction zone with the aid of a carrier gas.

13. The process according to any of claims 10 to 12, wherein the compound of the formula II is conducted through the reaction zone with a catalyst hourly space velocity of 0.01 to 5 kg of compound II per kg of catalyst and hour.

## Revendications

1. Procédé de fabrication d'arylpropènes de formule générale (I) 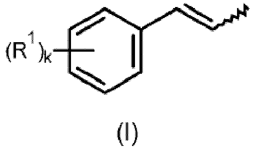 dans laquelle
k présente la valeur 0, 1, 2 ou 3, et
les R¹ sont choisis indépendamment les uns des autres parmi alcoxy en C₁-C₆, di-(alkyle en C₁-C₆)-amines et hydroxy,
comprenant la thermolyse d'au moins un composé de 1,1-diarylpropane de formule générale (II) 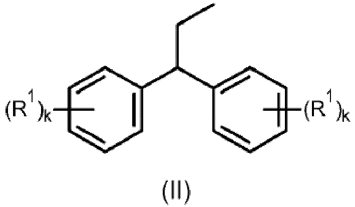 dans laquelle k et R¹ ont les significations susmentionnées,
le composé de formule II étant mis en contact dans la phase gazeuse dans une zone de réaction avec au moins un catalyseur oxydique acide solide.

2. Procédé selon la revendication 1, dans lequel, dans les formules I et II, k = 1 et R¹ représente hydroxy ou alcoxy en C₁-C₆ et notamment hydroxy ou méthoxy.

3. Procédé selon la revendication 1 ou 2, dans lequel les composés de 1,1-diarylpropane (II) comprennent au moins 50 % en moles, par rapport à la quantité totale des composés de formule II, de composés de formule générale (II.a) 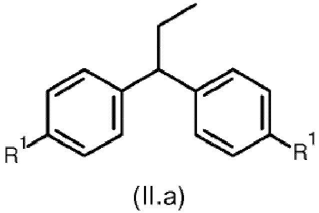 dans laquelle les deux R¹ sont identiques et ont les significations indiquées précédemment.

4. Procédé selon l'une quelconque des revendications précédentes, dans lequel au moins un solvant LM est en outre introduit dans la zone de réaction conjointement avec le composé de formule II.

5. Procédé selon la revendication 4, dans lequel le solvant LM est de l'eau.

6. Procédé selon la revendication 4 ou 5, dans lequel le rapport en masse entre le solvant LM et le composé de formule II se situe dans la plage allant de 20:1 à 1:100.

7. Procédé selon l'une quelconque des revendications précédentes, dans lequel le catalyseur comprend du dioxyde de silicium.

8. Procédé selon la revendication 7, dans lequel le catalyseur est une silice, qui est éventuellement imprégnée avec un ou plusieurs acides inorganiques.

9. Procédé selon l'une quelconque des revendications précédentes, dans lequel le composé de formule II est mis en contact avec le catalyseur à une température dans la plage allant de 250 à 650 °C.

10. Procédé selon l'une quelconque des revendications précédentes, dans lequel le composé de formule II est introduit en continu dans la zone de réaction qui contient le catalyseur oxydique solide.

11. Procédé selon la revendication 10, dans lequel le composé de formule II est introduit en continu dans la zone de réaction et le produit de réaction est refroidi directement après la sortie de la zone de réaction.

12. Procédé selon la revendication 10 ou 11, dans lequel le composé de formule II est introduit dans la zone de réaction à l'aide d'un gaz vecteur.

13. Procédé selon l'une quelconque des revendications 10 à 12, dans lequel le composé de formule II est introduit dans la zone de réaction avec un chargement du catalyseur de 0,01 à 5 kg de composé II par kg de catalyseur et par heure.
